# FASCICULE DE BREVET EUROPEEN

(11) **EP 0 582 502 B1**
(45) Date de publication et mention de la délivrance du brevet: **02.12.1998**
(21) Numéro de dépôt: 93401896.1
(22) Date de dépôt: 22.07.1993
(51) Int. Cl.: A61K 31/505, A61K 9/70

(54) **Préparation pharmaceutique transdermique à base d'alfuzosine**
Transdermal Alfuzosin enthaltendes Arztneimittel
Transdermal composition containing alfuzosin

(30) Priorité: 05.08.1992 FR 9209711
(43) Date de publication de la demande: 09.02.1994
(73) Titulaire: SYNTHELABO, 92350 Le Plessis Robinson (FR)
(72) Inventeur: Andrieu, Véronique, F-92100 Boulogne Billancourt (FR); Henrion, Louis, F-92100 Boulogne (FR); Montel, Jean, F-78400 Chatou (FR); Wick, Alexander, F-78860 St Nom la Bretèche (FR)
(74) Mandataire: Thouret-Lemaitre, Elisabeth

(56) Documents cités:
- EP-A- 0 204 597
- GB-A- 2 197 589

## Description

La présente invention a pour objet une préparation pharmaceutique transdermique contenant code principe actif, le N-[3-[(4-amino-6,7-dimethoxy-2-quinazolinyl)methylamino]propyl]tetrahydro-2-furanecarboxamide (ou alfuzosine) ou l'un de ses sels et l'obtention de cette préparation.

Plus spécifiquement, la présente invention a trait à une préparation pharmaceutique transdermique formulée de façon à permettre l'absorption de l'alfuzosine au travers de la peau en un site d'application soigneusement choisi.

En outre cette préparation permet une activité thérapeutique à effet prolongé.

L'alfuzosine est un composé connu, antagoniste sélectif des récepteurs alpha-1-adrénergiques post-synaptiques.

L'alfuzosine manifeste une spécificité pour les récepteurs alpha-1-adrénergiques situés au niveau du trigone vésical de l'urètre et de la prostate.

L'alfuzosine, sous forme de chlorhydrate, est utilisée en thérapeutique, en raison des caractéristiqus ci-dessus, pour traiter l'hypertrophie bénigne de la prostate. Le brevet GB-A-2 197 589 décrit l'utilisation de l'alfuzosine pour stimuler la pousse des cheveux et/ou en retarder la chute. Dans ce cas, l'alfuzosine est administrée topiquement sous forme de lotion ou de gel. Cependant, l'administration transdermique d'alfuzosine, ou une formulation comprenant l'alfuzosine et un promoteur d'absorption, ne sont pas décrites.
L'utilisation thérapeutique de l'alfuzosine nécessite une administration qui conduit à des taux plasmatiques suffisants, réguliers au cours du temps et maintenus le plus longtemps possible.

De manière générale, pour tout médicament, une élévation brutale du taux plasmatique ou un pic plasmatique trop élevé entraine des effets non favorables. Des taux plasmatiques trop faibles ou discontinus portent atteinte à l'efficacité thérapeutique. C'est pourquoi la recherche de taux plasmatiques satisfaisants conduit parfois à des administrations orales fréquentes ou à des administrations parentérales peu acceptées, complexes et coûteuses.
Dans chaque cas il s'ensuit une gêne qui porte atteinte à l'observance de la part du malade.

Pour éviter les inconvénients rappelés ci-dessus des formes orales et parentérales, il est très souvent recherché la possibilité de recourir à une administration transdermique. Ce type d'administration aboutit à des taux plasmatiques suffisants, réguliers, constants et prolongés. De plus l'administration est aisée, journalière ou plus espacée et facile à réaliser pour le malade et le médecin.

De par leur nature les molécules sont plus ou moins absorbées au travers de la peau humaine.
Pour faciliter le passage transdermique des molécules actives, plusieurs possibilités existent, telles que la modification de la perméabilité de la couche cornée, le passage par les follicules pileux, le changement d'état de la surface de la peau.

Divers moyens chimiques ou physiques peuvent également activer et faciliter le passage transdermique des molécules, par exemple l'emploi de promoteurs d'absorption, de courants électriques ou d'ondes sonores.

Les travaux scientifiques publiés à ce jour font ressortir qu'il n'y a pas de moyen universel, applicable à toutes les molécules, mais une adaptation indispensable à chaque molécule et à chacune de ses utilisations thérapeutiques.

Pour l'alfuzosine, la Demanderesse a procédé à une recherche expérimentale systématique des solvants, excipients et promoteurs d'absorption et tout particulièrement de leurs combinaisons qui contribuent à permettre l'amélioration du passage transdermique.

Cette recherche a été initialement effectuée sur un modèle "ex-vivo" utilisant de la peau humaine prélevée lors d'opérations de chirurgie plastique et soigneusement conservée pour garantir le maintien de ses propriétés physiologiques. Un tel modèle a été décrit par T.J. Franz (Current Problems in Dermatology, 7,58-68, 1978) et par H. Durrheim et coll. (Journal of Pharmaceutical Sciences, 69, 7, 781-786, 1980).

La Demanderesse a trouvé et démontré que certains solvants organiques et certains composés aliphatiques, et surtout leurs combinaisons optimales, permettent un passage transdermique suffisant de l'alfuzosine pour obtenir les taux plasmatiques qui répondent aux critères énumérés précédemment.

La présente invention a donc pour objet une préparation pharmaceutique transdermique contenant le N-[3-[(4-amino-6,7-diméthoxy-2-quinazolinyl)méthylamino]propyl]tetrahydro-2-furanecarboxamide ou l'un de ses sels et un mélange composé d'eau, d'un ou plusieurs solvants organiques, tels que l'éthanol, l'acétate d'éthyle ou le propylèneglycol, et d'un ou plusieurs promoteurs d'absorption, tels que des acides ou des alcools gras à chaîne aliphatique de 5 à 30 atomes de carbone.

Ces alcools et acides aliphatiques peuvent être saturés, insaturés, ramifiés ou substitués. Il s'agit par exemple et non exhaustivement des composés valériques, capryliques, isovalériques, caproïques, oenanthiques, pélargoniques, capriques, lauriques, myristiques, palmitiques, margariques, stéariques, oléiques, arachidiques, beheniques, lignoceriques, ceratiques, montaniques, melissiques, etc... et de leurs homologues insaturés ou ramifiés ou encore substitués.

Selon l'invention, les composés préférés sont l'acide oléique, caprylique ou caprique.

Les promoteurs d'absorption peuvent être utilisés seuls ou en association, dans une proportion de 1 à 500 % du poids de l'alfuzosine.

La teneur en alfuzosine peut aller de 5 à 100 mg par préparation.

D'autres excipients peuvent être ajoutés à la préparation de façon à aboutir à la forme pharmaceutique désirée, telle que pommade, crème, gel, émulsion, dispositif ou timbre transdermique, etc...

Enfin il peut être nécessaire d'introduire dans la préparation un composé corrigeant certains effets secondaires locaux de la préparation. On peut utiliser en plus, par exemple, des agents hydratants (urée, sorbitol ou polyols similaires), des agents apaisants (anesthésiques locaux par exemple), des agents anti-inflammatoires (corticoïdes ou AINS) ou des antihistaminiques.

Le dispositif transdermique peut être constitué par un système matriciel, un système réservoir ou un système constitué d'enductions successives. Dans tous les cas, le système inclut l'alfuzosine ou l'un de ses sels, le ou les promoteur(s), les excipients technologiques et le ou les composés correcteurs décrits ci-dessus.
Le dispositif transdermique peut en outre inclure des constituants propres à réaliser le système, à assurer sa conservation et à permettre son utilisation.

Ces constituants peuvent être scindés en trois groupes : supports passifs, constituants actifs du système, adhésifs.

Le support passif peut être un film métallique, par exemple d'aluminium, un tissu ou un réseau non tissé de fibres naturelles ou artificielles, un film polymérique tel que polyéthylène, polypropylène, polytétrafluoro-éthylène, polymère cellulosique, acrylique, vinylique, silicone, acrylonitrile etc...

Les constituants actifs du système peuvent être des films ou des matrices polymériques tels que : polyéthylène, polypropylène, polytetrafluoro-éthylène, polymère cellulosique, acrylique, vinylique, silicone, acrylonitrile etc...

Le ou les adhésifs peuvent être constitués de caoutchouc naturel ou synthétique, polyisobutylène, polyacrylates, polyvinyléther etc...

La préparation pharmaceutique transdermique d'alfuzosine selon l'invention est caractérisée par le fait qu'après son application sur la peau humaine, les effets thérapeutiques de l'alfuzosine sont obtenus de façon stable, continue et prolongée.

Selon l'invention on peut obtenir les préparations transdermiques de la manière suivante :

On mélange du propylèneglycol et de l'acide oléique dans les proportions telles que leur teneur totale représente 4 à 15 % (m/m) de la préparation finale.

On disperse ce mélange sous vide d'air et forte agitation dans 4 à 9 % (m/m) d'eau ultra-pure, puis on incorpore 2 à 7 % (m/m) d'un viscosifiant (silice colloïdale, par exemple).

Cette préémulsion est alors mélangée à une dispersion aqueuse d'un copolymère neutre à base de polyacrylates et de polyméthacrylates, sous vide d'air et à faible agitation, dans la proportion de 59 à 89,5 % (m/m).

L'alfuzosine (base) est mise en suspension dans la préparation à raison de 0,5 à 10 % (m/m), sous vide d'air et sous très forte agitation.

La préparation est étalée sur un film d'aluminium écroui (d'épaisseur 30 µm, par exemple) enduit de polyéthylène sur la face recevant la préparation, de telle sorte que pour une surface donnée, le volume de préparation contienne une quantité de principe actif suffisante à sa cinétique de libération.

On sèche et polymérise en étuve ventilée.

La préparation est enduite d'une colle de polyaminoacrylate d'acides gras en solution aqueuse, en quantité suffisante pour assurer une adhésion convenable pendant 24 heures.

On sèche en étuve ventilée.

Un film protecteur est appliqué sur la face collante.

Les systèmes transdermiques sont conditionnés dans des sachets scellés imperméables et opaques.

## Revendications

1. Préparation pharmaceutique destinée à la voie transdermique, caractérisée en ce qu'elle contient de l'alfuzosine [(N-[3-[(4-amino-6,7-diméthoxy-2-quinazolinyl)methylamino]propyl]tetrahydro-2-furanecarboxamide)] ou l'un de ses sels et un mélange composé d'eau, d'un ou plusieurs solvants organiques et d'un ou plusieurs promoteurs d'absorption.

2. Préparation pharmaceutique selon la revendication 1, caractérisée par le fait que le ou/les solvants organiques est(sont) choisi(s) parmi l'éthanol, l'acétate d'éthyle ou le propylèneglycol.

3. Préparation pharmaceutique selon la revendication 1 ou la revendication 2, caractérisée par le fait que le(ou les) promoteurs d'absorption est(sont) choisi(s) parmi les acides et/ou alcools gras à chaîne aliphatique de 5 à 30 atomes de carbone.

4. Préparation pharmaceutique destinée à la voie transdermique selon l'une quelconque des revendications 1 à 3, caractérisée par le fait que la proportion de promoteurs d'absorption est de 1 à 500 % du poids d'alfuzosine.

5. Préparation pharmaceutique destinée à la voie transdermique selon l'une quelconque des revendications 1 à 4, caractérisée par le fait que la quantité d'alfuzosine peut aller de 5 à 100 mg, par dose unitaire.

## Claims

1. Pharmaceutical preparation intended for the transdermal route, characterized in that it contains alfuzosin [N-[3-[(4-amino-6,7-dimethoxy-2-quinazolinyl)-methylamino]propyl]tetrahydro-2-furancarboxamide] or one of its salts and a mixture composed of water, one or a number of organic solvents and one or a number of absorption promoters.

2. Pharmaceutical preparation according to Claim 1, characterized in that the organic solvent(s) is (are) chosen from ethanol, ethyl acetate or propylene glycol.

3. Pharmaceutical preparation according to Claim 1 or Claim 2, characterized in that the absorption promoter(s) is (are) chosen from fatty acids and/or alcohols containing an aliphatic chain with 5 to 30 carbon atoms.

4. Pharmaceutical preparation intended for the transdermal route according to any one of Claims 1 to 3, characterized in that the proportion of absorption promoters is from 1 to 500 % of the weight of alfuzosin.

5. Pharmaceutical preparation intended for the transdermal route according to any one of Claims 1 to 4, characterized in that the amount of alfuzosin can range from 5 to 100 mg per unit dose.

## Patentansprüche

1. Pharmazeutische Zubereitung zur Verabreichung auf transdermalem Wege, dadurch gekennzeichnet, daß sie Alfuzosin [(N-[3-[(4-Amino-6,7-dimethoxy-2-chinazolinyl)-methylamino]-propyl]-tetrahydro-2-furancarboxamid)] oder eines seiner Salze und eine Mischung aus Wasser, einem oder mehreren organischen Lösungsmitteln und einem oder mehreren Absorptionspromotoren enthält.

2. Pharmazeutische Zubereitung nach Anspruch 1, dadurch gekennzeichnet, daß das und/oder die organischen Lösungsmittel aus Ethanol, Ethylacetat oder Propylenglykol ausgewählt ist (sind).

3. Pharmazeutische Zubereitung nach Anspruch 1 oder Anspruch 2, dadurch gekennzeichnet, daß der (oder die) Absorptionspromotor(en) aus Fettsäuren und/oder Fettalkoholen mit einer aliphatischen Kette mit 5 bis 30 Kohlenstoffatomen ausgewählt ist (sind).

4. Pharmazeutische Zubereitung zur Verabreichung auf transdermalem Wege nach irgendeinem der Ansprüche 1 bis 3, dadurch gekennzeichnet, daß der Anteil an Absorptionspromotoren 1 bis 500 % des Gewichts von Alfuzosin beträgt.

5. Pharmazeutische Zubereitung zur Verabreichung auf transdermalem Wege nach irgendeinem der Ansprüche 1 bis 4, dadurch gekennzeichnet, daß die Alfuzosin-Menge 5 bis 100 mg pro Einheitsdosis betragen kann.
